(19) Europäisches Patentamt
European Patent Office
Office européen des brevets

(11) EP 3 937 900 B1

(12) # EUROPEAN PATENT SPECIFICATION

(45) Date of publication and mention of the grant of the patent:
**02.08.2023 Bulletin 2023/31**

(21) Application number: **20716675.2**

(22) Date of filing: **13.03.2020**

(51) International Patent Classification (IPC):
***A61K 8/73*** *(2006.01)* ***A61K 8/58*** *(2006.01)*
***A61K 8/46*** *(2006.01)* ***A61K 8/41*** *(2006.01)*
***A61Q 5/02*** *(2006.01)* ***A61Q 5/12*** *(2006.01)*

(52) Cooperative Patent Classification (CPC):
**A61K 8/731; A61K 8/416; A61K 8/463; A61K 8/585; A61Q 5/02; A61Q 5/12;**
A61K 2800/48; A61K 2800/5426; A61K 2800/594; A61K 2800/85

(86) International application number:
**PCT/US2020/022577**

(87) International publication number:
**WO 2020/186147 (17.09.2020 Gazette 2020/38)**

(54) **ENHANCED SILICON DEPOSITION COMPOSITIONS AND METHOD THEREFORE**

VERBESSERTE SILIZIUMABSCHEIDUNGSZUSAMMENSETZUNGEN UND VERFAHREN DAFÜR

COMPOSITIONS DE DÉPÔT DE SILICIUM AMÉLIORÉ ET MÉTHODE CORRESPONDANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.03.2019 US 201962818377 P**

(43) Date of publication of application:
**19.01.2022 Bulletin 2022/03**

(73) Proprietor: **Lubrizol Advanced Materials, Inc.**
**Cleveland, OH 44141-3247 (US)**

(72) Inventors:
- **LI, Wing, K.**
**Piscataway, NJ 08854 (US)**
- **TAMARESELVY, Krishnan**
**Piscataway, NJ 08854 (US)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-2008/057985** **WO-A1-2008/079693**
**WO-A2-2012/019934** **JP-A- 2009 040 968**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 937 900 B1

## Description

### FIELD OF THE INVENTION

The disclosed technology relates to stable conditioning shampoos comprising silicone oil and a fermentation derived cellulose (FDC) component. The FDC component provides good suspension of particulate and insoluble materials such as aesthetic beads and mica in surfactant-based systems, as well as enhances the deposition of silicone conditioning agents to keratinous substrates such as the hair and skin.

### BACKGROUND

Most synthetic rheology modifiers (e.g., Carbomers and acrylic copolymers) used in typical cleansing systems have a negative impact on silicone deposition, especially small silicone particle size or small water insoluble silicone droplets which deleteriously affects the conditioning performance on the hair. It has been discovered that fermentation derived cellulose (FDC) provides good suspension of particulate and insoluble materials such as aesthetic beads and mica in surfactant-based systems, as well as enhances the deposition of silicone conditioning agents to keratinous substrates such as the hair and skin. Fermentation derived cellulose is a bio-based material with good surfactant compatibility. It has been found that FDC is a useful silicone conditioning agent deposition aid which can be formulated into 2-in-1 shampoo.

Silicone deposition from a conditioning and cleansing composition can be influenced either negatively or positively by the type of thickener or rheology modifier used in the formulation. It is known that synthetic acrylate copolymers tend to decrease silicone deposition. Consequently, less than an optimal amount of silicone conditioning agent is released to the hair and the conditioning benefit is diminished. There remains a need for a stabilizer system that provides the desired rheological properties (e.g., thickening, shear thinning, flow and suspension of particulate materials), stabilization and deposition of silicone conditioning agents with acceptable conditioning cues to consumers.

### BRIEF DESCRIPTION OF THE DRAWINGS

Fig. 1 is a graph comparing silicone deposition on non-damaged European hair from conditioning shampoo formulations containing various rheology modifying polymers.

### DESCRIPTION

Surprisingly, it has been discovered that the use of FDC and blends with other polysaccharides such as xanthan gum, guar gum, carboxymethylcellulose as described in the US 10214708 B2 when employed as thickeners/stabilizers in a conditioning and cleansing composition (including anionic and/or amphoteric surfactants) containing cationic polymers such as disclosed in paragraphs [0211]-[0218] of US 2016/0317424 provide multiple benefits including stable silicone containing cleansing compositions, enhanced deposition of the silicone material onto keratinous substrates, rich creamy foam profile, and acceptable conditioning benefits.

FDC can be obtained from a Sphingomonas ferment extract (commercially available as Kelco Care™ Diutan Gum) is the extract of the product obtained by the fermentation of Sphingomonas. Kelco Care™ Diutan Gum is a natural high molecular weight polysaccharide with a low anionic charge density produced by fermentation of the mircrorganism, Sphingomonas sp. ATCC 53159. It is comprised of six sugar units of d-glucose, d-glucuronic acid, d-glucose (with 2 l-rhamnose in the side chain) and l-rhamnoses, forming a linear backbone with a repeating side chain. Diutan gum has high molecular weight (typically millions of kD) and thus long molecular chain length. This leads to diutan gum polymer chain entanglement at relatively low concentrations in solution. The structured network of entangled, stiff molecules creates high viscosity at low shear rates, resulting in outstanding suspension properties. The molecules in the complex network of a diutan gum solution are weakly associated. This network is progressively disrupted under the influence of applied shear stress making diutan gum solutions highly pseudoplastic. This rheology behavior makes Sphingomonas Ferment Extract (Kelco Care™ Diutan Gum) a robust candidate as a thickener and stabilizing agent in challenging cosmetic formulations such as low or high pH, high ion contents or natural formulations.

Microfibrous cellulose can be prepared by mechanically disrupting/altering cereal, wood, or cotton-based cellulose fibers, and is commercially available under the Betafib™ trade name supplied by Royal Cosun.

The FDC can be employed in the 2-in-1 shampoo compositions in an amount ranging from about 0.05 to about 5 wt.%, or from about 0.1 to about 3 wt.%, or from about 0.5 to about 2 wt.% based on the total weight of the composition.

Auxiliary Natural Polymers and Gums

Auxiliary natural polymers and gums can be formulated into the 2-in-1 conditioning compositions. Natural polymers and gums include, but are not limited to Polysaccharides obtained from tree and shrub exudates, such as gum Arabic, gum gahatti, and gum tragacanth, as well as pectin; seaweed extracts, such as alginates and carrageenans; algae extracts, such as agar; microbial polysaccharides, such as xanthan, gellan, and wellan; cellulose ethers, such as ethylhexylethylcellulose, hydroxybutylmethylcellulose, hydroxyethylmethylcellulose, hydroxypropylmethylcellulose, methylcellulose, carboxymethylcellulose, carboxyethyl cellulose, hydroxyethylcellulose, and hydroxypropylcellulose; polygalactomannans, such as fenugreek gum, cassia gum, locust bean gum, tara gum, and guar gum; and cationic derivatives thereof.

The auxiliary natural polymers and gums can be present in an amount ranging from about 0.1 to about 3 wt.% or from about 0.25 to about 2 wt.%, or from about 0.5 to about 1 wt.%, based on the total wt. of the composition.

Silicone Conditioners

The silicone conditioning agent may comprise volatile silicones, non-volatile silicones, water soluble silicones (e.g., dimethicone copolyols), and mixtures thereof. If volatile silicones are present, they are typically employed as a solvent or carrier for commercially available forms of non-volatile silicone fluid conditioning agents such as oils and gums and resins. Volatile silicone fluids are often included in the conditioning package to improve silicone fluid deposition efficacy or to enhance the shine, sheen or glossiness of the hair. Volatile silicone materials are frequently included in formulations to enhance sensory attributes (e.g., feel) on the hair, scalp and skin.

In one aspect, the silicone conditioning agent is non-volatile and includes silicone oils, gums, resins and mixtures thereof. By non-volatile is meant that the silicone has a very low vapor pressure at ambient temperature conditions (e.g., less than 2 mm Hg at 20°C). The non-volatile silicone conditioning agent has a boiling point above about 250°C in one aspect, above about 260°C in another aspect, and above about 275°C in a further aspect. Background information on silicones including sections discussing silicone oils, gums, and resins, as well as their manufacture, are found in Encyclopedia of Polymer Science and Engineering, vol. 15, 2d ed., pp 204-308, John Wiley & Sons, Inc. (1989).

In one aspect, the silicone oil conditioning agent includes amino functional and quaternized polyorganosiloxanes represented by the formula:

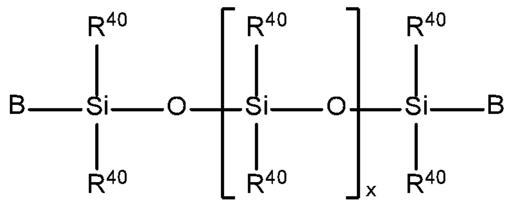

wherein B independently represents hydroxy, methyl, methoxy, ethoxy, propoxy, and phenoxy; $R^{40}$ independently represents methyl, ethyl, propyl, phenyl, methylphenyl, phenylmethyl, a primary, secondary or tertiary amine, a quaternary group selected from a group selected from:

$-R^{41}-N(R^{42})CH_2CH_2N(R^{42})_2$;

$-R^{41}-N(R^{42})_2$;

$-R^{41}-N^+(R^{42})_3CA^-$; and

$-R^{41}-N(R^{42})CH_2CH_2N^+(R^{42})H_2\ CA^-$

wherein $R^{41}$ is a linear or branched, hydroxyl substituted or unsubstituted alkylene or alkylene ether moiety containing 2 to 10 carbon atoms; $R^{42}$ independently is hydrogen, $C_1$-$C_{20}$ alkyl, phenyl or benzyl; $CA^-$ is a halide ion selected from chlorine, bromine, iodine and fluorine; and the sum of m+n ranges from about 7 to about 1000 in one aspect, from about 50 to about 250 in another aspect, and from about 100 to about 200 in another aspect, subject to the proviso that m or n is not 0. In one aspect, B is hydroxy and $R^{40}$ is $-(CH_2)_3NH(CH_2)_3NH_2$. In another aspect, B is methyl and $R^{40}$ is $-(CH_2)_3NH(CH_2)_3NH_2$. In still another aspect, B is methyl and $R^{40}$ is a quaternary ammonium moiety represented by $-(CH_2)_3OCH_2CH(OH)CH_2N^+(R^{42})_3\ CA^-$; wherein $R^{42}$ and $CA^-$ are as previously defined.

The silicone oil conditioning agents can have a viscosity ranging from about above about 25 to about 1,000,000

mPa·s at 25°C in one aspect, from about 100 to about 600,000 mPa·s in another aspect, and from about 1000 to about 100,000 mPa·s still another aspect, from about 2,000 to about 50,000 mPa·s in yet another aspect, and from about 4,000 to about 40,000 mPa·s in a further aspect. The viscosity is measured by means of a glass capillary viscometer as described by Dow Corning Corporate Test Method CTM004, dated July 20, 1970. In one aspect the silicone oils have an average molecular weight below about 200,000 daltons. The average molecular weight can typically range from about 400 to about 199,000 daltons in one aspect, from about 500 to about 150,000 daltons in another aspect, from about 1,000 to about 100,000 daltons in still another aspect, from about 5,000 to about 65,000 daltons in a further aspect.

Exemplary silicone oil conditioning agents include, but are not limited to, polydimethylsiloxanes (dimethicones), polydiethylsiloxanes, polydimethyl siloxanes having terminal hydroxyl groups (dimethiconols), polymethylphenylsiloxanes, phenylmethylsiloxanes, amino functional polydimethylsiloxanes (amodimethicones), and mixtures thereof.

In addition to the quaternized silicone conditioning agents set forth under the formulas above, suitable non-limiting examples (which may or may not fall under the above formulas are Silicone Quaternium-1, Silicone Quaternium-2, Silicone Quaternium-2 Panthenol Succinate, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-16/Glycidoxy Dimethicone Crosspolymer, Silicone Quaternium-17, Silicone Quaternium-18, Silicone Quaternium-20, Silicone Quaternium-21 and Quaternium-80.

Another silicone conditioning agent useful in the disclosed technology is a silicone gum. A silicone gum is a polyorganosiloxane material of the same general structure of the silicone oils set forth above wherein B independently represents hydroxy, methyl, methoxy, ethoxy, propoxy, and phenoxy; $R^{40}$ independently represents methyl, ethyl, propyl, phenyl, methylphenyl, phenylmethyl, and vinyl. Silicone gums have a viscosity measured at 25°C of greater than 1,000,000 mPa·s. The viscosity can be measured by means of a glass capillary viscometer as described above for the silicone oils. In one aspect, the silicone gums have an average molecular weight about 200,000 daltons and above. The molecular weight can typically range from about 200,000 to about 1,000,000 daltons. It is recognized that the silicone gums described herein can also have some overlap with the silicone oils described previously. This overlap is not intended as a limitation on any of these materials.

Suitable silicone gums for use in the silicone component of compositions of the disclosed technology are polydimethylsiloxanes (dimethicones), optionally having terminal end groups such as hydroxyl (dimethiconols), polymethylvinylsiloxane, polydiphenylsiloxane, and mixtures thereof.

Silicone resins can be included as a silicone conditioning agent suitable for use in the compositions of the disclosed technology. These resins are crosslinked polysiloxanes. The crosslinking is introduced through the incorporation of trifunctional and tetrafunctional silanes with monofunctional and/or difunctional silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units (and hence, a sufficient level of crosslinking) such that they form a rigid or hard film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. In one aspect, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinyl-chlorosilanes, and terachlorosilane, with the methyl substituted silanes being most commonly utilized.

Silicone materials and silicone resins can be identified according to a shorthand nomenclature system known to those of ordinary skill in the art as "MDTQ" nomenclature. Under this naming system, the silicone is described according to the presence of various siloxane monomer units which make up the silicone. The "MDTQ" nomenclature system is described in the publication entitled "Silicones: Preparation, Properties and Performance"; Dow Corning Corporation, 2005, and in U.S. Patent. No. 6,200,554.

Exemplary silicone resins for use in the compositions of the disclosed technology include, but are not limited to MQ, MT, MTQ, MDT and MDTQ resins. In one aspect, methyl is the silicone resin substituent. In another aspect, the silicone resin is selected from a MQ resins, wherein the M:Q ratio is from about 0.5:1.0 to about 1.5:1.0 and the average molecular weight of the silicone resin is from about 1000 to about 10,000 daltons.

The optional volatile silicones referred to above include linear polydimethylsiloxanes and cyclic polydimethylsiloxanes (cyclomethicones), and mixtures thereof. Volatile linear polydimethylsiloxanes (dimethicones) typically contain about 2 to about 9 silicon atoms, alternating with oxygen atoms in a linear arrangement. Each silicon atom is also substituted with two alkyl groups (the terminal silicon atoms are substituted with three alkyl groups), such as, for example, methyl groups. The cyclomethicones typically contain about 3 to about 7 dimethyl substituted silicon atoms in one aspect and from about 3 to about 5 in another aspect, alternating with oxygen atoms, in a cyclic ring structure. The term "volatile" means that the silicone has a measurable vapor pressure, or a vapor pressure of at least 2 mm of Hg at 20°C. The volatile silicones have a viscosity of 25 mPa·s or less at 25°C in one aspect, from about 0.65 about to about 10 mPa·s

in another aspect, from about 1 to about 5 mPa·s in still another aspect, and from about 1.5 to about 3.5 mPa·s in a further aspect. A description of linear and cyclic volatile silicones is found in Todd and Byers, "Volatile Silicone Fluids for Cosmetics", Cosmetics and Toiletries, Vol. 91(1), pp. 27-32 (1976), and in Kasprzak, "Volatile Silicones", Soap/Cosmetics/Chemical Specialties, pp. 40-43 (December 1986).

Exemplary volatile linear dimethicones include, but are not limited to, hexamethyldisiloxane, octamethyltrisiloxane, decamethyltetrasiloxane, dodecamethylpentasiloxane and blends thereof. Volatile linear dimethicones and dimethicone blends are commercially available from Dow Corning Corporation as Dow Corning 200® Fluid (e.g., product designations 0.65 CST, 1 CST, 1.5 CST, and 2 CST) and Dow Corning® 2-1184 Fluid. xemplary volatile cyclomethicones are D4 cyclomethicone (octamethylcyclotetrasiloxane), D5 cyclomethicone (decamethylcyclopentasiloxane), D6 cyclomethicone, and blends thereof (e.g., D4/D5 and D5/D6). Volatile cyclomethicones and cyclomethicone blends are commercially available from Momentive Performance Materials Inc. as SF1173, SF1202, SF1256, and SF1258 silicone fluids, and Dow Corning Corporation as Dow Corning® 244, 245, 246, 345, and 1401 silicone fluids. Blends of volatile cyclomethicones and volatile linear dimethicones also can be employed.

Another class of silicone conditioning agent useful in the disclosed technology is a dimethicone copolyol. The dimethicone copolyols are linear or branched copolymers of dimethylsiloxane (dimethicone) modified with terminal and/or pendant alkylene oxide units. Suitable alkylene oxide units are selected from ethylene oxide, propylene oxide, butylene oxide, and combinations thereof. When mixtures of alkylene oxides are present, the alkylene oxide units can be arranged as random or block segments. Dimethicone copolyols can be water soluble or oil soluble depending on the amount and type of polyalkylene oxide present in the dimethicone polymer. The dimethicone copolyols can be derivatized to be anionic, cationic, amphoteric or nonionic in character.

In one aspect, the nonionic dimethicone copolyol contains pendant polyoxyalkylene moieties and can be represented by the formula:

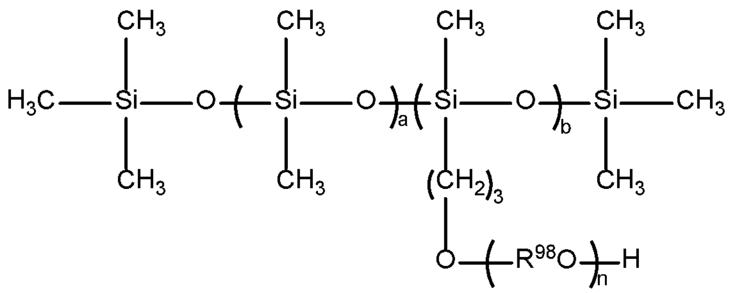

wherein a represents an integer ranging from about 0 or 1 to about 500; b is an integer ranging from about 1 to about 100; $(R^{98}O)_n$ is a polyoxyalkylene moiety which can be arranged as a homopolymer, a random copolymer, or a block copolymer of oxyalkylene units; $R^{98}$ is a divalent alkylene moiety selected from $C_2H_4$, $C_3H_6$, or $C_4H_8$, and combinations thereof; and n is an integer ranging from about 1 to about 50 in one aspect, from about 3 to about 35 in another aspect, from about 5 to about 25 in a still another aspect, and from about 8 to about 20 in a further aspect.

Exemplary nonionic dimethicone copolyols containing pendant polyoxyalkylene moieties are commercially available under the Silsoft® trade name from Momentive Performance Materials. Specific product designations include, but are not limited to, Silsoft product designations 430 and 440 (PEG/PPG 20/23 Dimethicone), 475 (PEG/PPG 20/6 Dimethicone), 805 (PEG-8 Dimethicone), 875 and, 880 (PEG-12 Dimethicone), 895 (PEG-17 Dimethicone), and 910 (PPG-12 Dimethicone). Other commercially available dimethicone copolyols include Silsense™ Copolyol-1 a dimethicone copolyol blend (PEG-33 Dimethicone and PEG-8 Dimethicone and PEG-14 Dimethicone) from Lubrizol Advanced Materials, Inc.

In another aspect, the nonionic dimethicone copolyol contains terminal polyoxyalkylene moieties and can be represented by the formula:

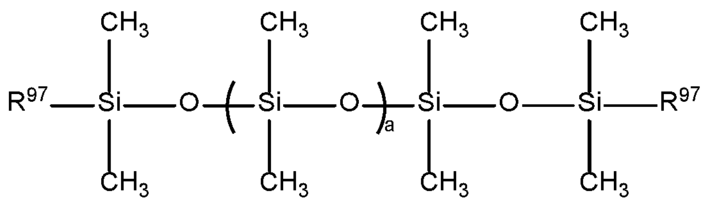

wherein $R^{97}$ independently is selected from methyl and the radical $-(CH_2)_m-O-(R^{98}O)_n-H$; a is an integer ranging from

about 1 to about 500; $(R^{98}O)_n$ is a polyoxyalkylene moiety which can be arranged as a homopolymer, a random copolymer, or a block copolymer of oxyalkylene units; $R^{98}$ is a divalent alkylene moiety selected from $C_2H_4$, $C_3H_6$, or $C_4H_8$, and combinations thereof; m is an integer ranging from about 1 to about 5; and n is an integer ranging from about 1 to about 50 in one aspect, from about 3 to about 35 in another aspect, from about 5 to about 25 in a still another aspect, and from about 8 to about 20 in a further aspect; subject to the proviso that $R^{97}$ cannot both be methyl at the same time.

Exemplary nonionic dimethicone copolyols containing a terminal polyoxyalkylene moietie(s) also are commercially available under the Silsoft® trade name from Momentive Performance Materials under product designations 810 (PEG-8 Dimethicone), 860 (PEG-10 Dimethicone), 870 (PEG-12 Dimethicone), and 900 (PPG-12 Dimethicone).

In one aspect, the nonionic dimethicone copolyol contains esterified pendant polyoxyalkylene moieties and can be represented by the formula:

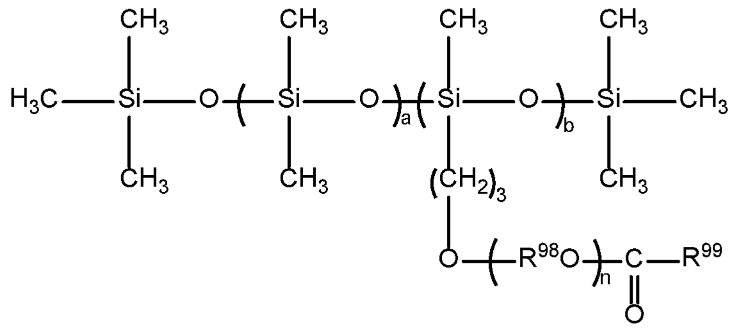

wherein a represents an integer ranging from about 0 or 1 to about 500; b is an integer ranging from about 1 to about 100; $(R^{98}O)_n$ is a polyoxyalkylene moiety which can be arranged as a homopolymer, a random copolymer, or a block copolymer of oxyalkylene units; $R^{98}$ is a divalent alkylene moiety selected from $C_2H_4$, $C_3H_6$, or $C_4H_8$, and combinations thereof; and n is an integer ranging from about 1 to about 50 in one aspect, from about 3 to about 35 in another aspect, from about 5 to about 25 in a still another aspect, and from about 8 to about 20 in a further aspect; and $R^{99}$ is $C_1$ to $C_{21}$ alkyl. In one aspect the acyl radical $-C(O)R^{99}$ that terminates the polyoxyalkylene moiety is derived from a saturated or unsaturated carboxylic or fatty acid obtained from natural or synthetic sources. These acids can be linear or branched. Suitable acids are selected from, but are not limited to caproic acid, enanthic acid, caprylic acid, pelargonic acid, capric acid, undecanoic acid lauric acid, myristic acid, palmitic acid, stearic acid, isosteric acid, oleic acid, linoleic acid, ricinoleic acid, and behenic acid which are typically obtained by hydrolyzing vegetable oils and animal oils such as coconuts oils, palm oil, tallow, linseed oil and soybean oil.

Dimethicone copolyol esters and methods for their preparation are disclosed in U.S. Patent No. 5,136,063, which is herein incorporated by reference. Exemplary dimethicone copolyol esters are commercially available under the Silsence™ trade name as product designations SW-12 (Dimethicone PEG-7 Cocoate) and DW-18 (Dimethicone PEG-7 isosterate) from Lubrizol Advanced Materials, Inc.

Other useful dimethicone copolyol esters contain at least one terminal polyoxyalkylene ester moiety as described in U.S. Patent No. 5,180,843, which is herein incorporated by reference.

In one aspect, the dimethicone copolyol contains a quaternium moiety and can be represented by the formula:

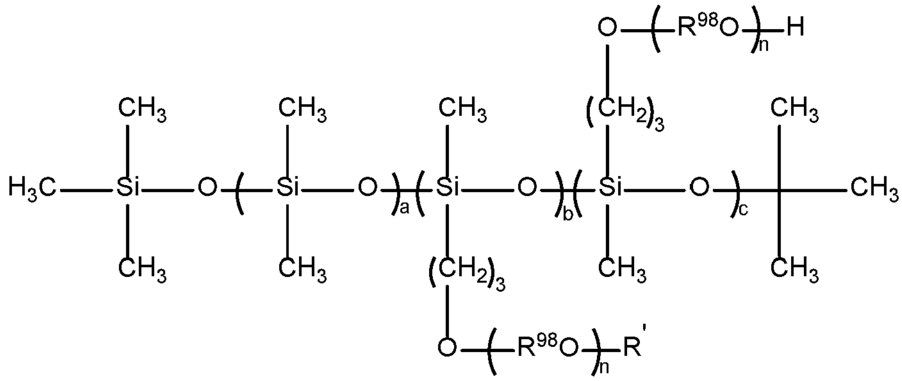

wherein a represents an integer ranging from about 0 or 1 to about 200; b is an integer ranging from about 1 to about 100; c is an integer ranging from about 0 or 1 to about 200; $(R^{98}O)_n$ is a polyoxyalkylene moiety which can

be arranged as a homopolymer, a random copolymer, or a block copolymer of oxyalkylene units; $R^{98}$ is a divalent alkylene moiety selected from $C_2H_4$, $C_3H_6$, or $C_4H_8$, and combinations thereof; and n independently is an integer ranging from about 1 to about 50 in one aspect, from about 3 to about 35 in another aspect, from about 5 to about 25 in a still another aspect, and from about 8 to about 20 in a further aspect; R' is selected from a radical of the following formulas:

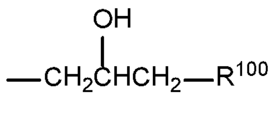

or

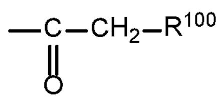

wherein $R^{100}$ is a quaternary nitrogen containing moiety selected from the formulas:

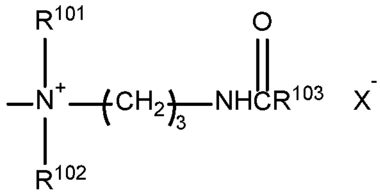

or

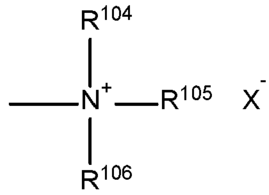

wherein $R^{101}$ and $R^{102}$ independently are selected from methyl or ethyl; $R^{103}$ is a $C_5$ to $C_{21}$ alkyl group; $R^{104}$, $R^{105}$, $R^{106}$ independently represent $C_1$ to $C_{20}$ alkyl; and X- is a salt forming anion. In one aspect, $R^{101}$ and $R^{102}$ are both methyl or both ethyl and $R^{103}$ is a $C_{11}$ to $C_{21}$ alkyl group. In one aspect, two of $R^{104}$, $R^{105}$ or $R^{106}$ are methyl and the remaining $R^{104}$, $R^{105}$ or $R^{106}$ that is not methyl is selected from a $C_{12}$ to $C_{20}$ alkyl group. In one aspect, X- is a chloride anion.

Dimethicone copolyol quaternary nitrogen containing compounds are disclosed in U.S. Patent Nos. 5,098,979 and 5,166,297, the disclosures of which are herein incorporated by reference. A suitable commercially available dimethicone copolyol quaternary compound (Silicone Quaternium-8) is available under the Silsence™ Q-Plus trade name from Lubrizol Advanced Materials, Inc.

Other exemplary quaternary nitrogen containing silicones that are useful in the disclosed technology Are Quaternium-80, Silicone Quaternium-1, Silicone Quaternium-2, Silicone Quaternium-2 Panthenol Succinate, Silicone Quaternium-3, Silicone Quaternium-4, Silicone Quaternium-5, Silicone Quaternium-6, Silicone Quaternium- 7, Silicone Quaternium-9, Silicone Quaternium-10, Silicone Quaternium-11 , Silicone Quaternium-12, Silicone Quaternium-15, Silicone Quaternium-16, Silicone Quaternium-16/Glycidoxy Dimethicone Crosspolymer, Silicone Quaternium-17, Silicone Quaternium-18, Silicone Quaternium-20 and Silicone Quaternium-21.

In one aspect, the dimethicone copolyol contains an amine functional group. Amine functional dimethicone copolyols can be represented by the formula:

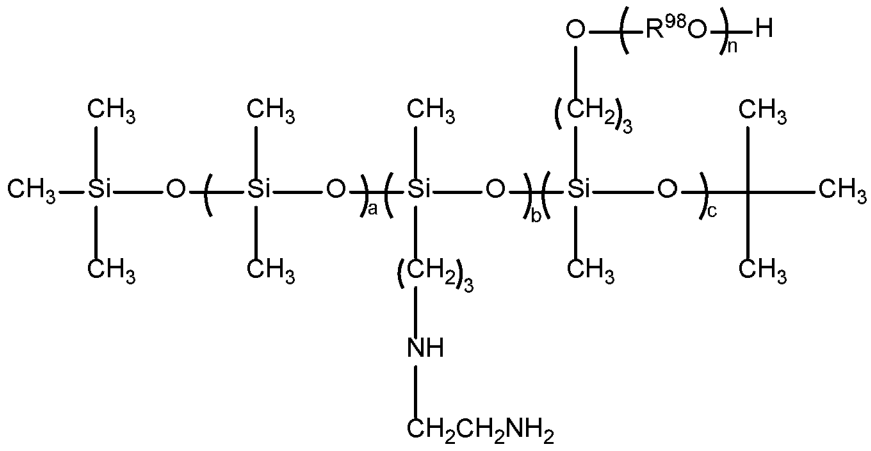

wherein a represents an integer ranging from about 0 or 1 to about 200; b is an integer ranging from about 1 to about

100; c is an integer ranging from about 1 to about 200; $(R^{98}O)_n$ is a polyoxyalkylene moiety which can be arranged as a homopolymer, a random copolymer, or a block copolymer of oxyalkylene units; $R^{98}$ is a divalent alkylene moiety selected from $C_2H_4$, $C_3H_6$, or $C_4H_8$, and combinations thereof; and n independently is an integer ranging from about 1 to about 50 in one aspect, from about 3 to about 35 in another aspect, from about 5 to about 25 in a still another aspect, and from about 8 to about 20 in a further aspect. A commercially available amine functional dimethicone copolyol is marketed under the trade name Silsense™ A-21 silicone (PEG-7 Amodimethicone) by Lubrizol Advanced Materials, Inc.

In one aspect, the dimethicone copolyol contains phosphate ester functionality. These compounds can be represented by the formula:

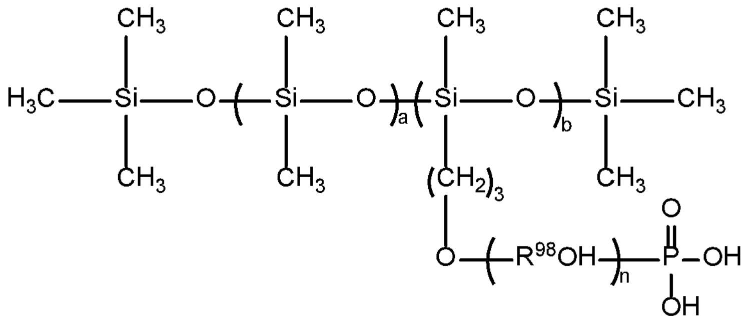

wherein a represents an integer ranging from about 0 or 1 to about 500; b is an integer ranging from about 1 to about 100; $(R^{98}O)_n$ is a polyoxyalkylene moiety which can be arranged as a homopolymer, a random copolymer, or a block copolymer of oxyalkylene units; $R^{98}$ is a divalent alkylene moiety selected from $C_2H_4$, $C_3H_6$, or $C_4H_8$, and combinations thereof; and n is an integer ranging from about 1 to about 50 in one aspect, from about 3 to about 35 in another aspect, from about 5 to about 25 in a still another aspect, and from about 8 to about 20 in a further aspect. A commercially available amine functional dimethicone copolyol is marketed under the trade name Silsense PE-100 silicone (Dimethicone PEG-8 Phosphate) by Lubrizol Advanced Materials, Inc.

The amount of silicone conditioner(s) in the compositions of the present technology should be sufficient to provide the desired conditioning performance to the hair, and generally ranges from about 0.01 to about 20 wt. % in one aspect, from about 0.05 to about 15 wt. % in another aspect, from about 0.1% to about 10 wt. % in still another aspect, and from about 1 to about 5 wt. % in a further aspect, based on the total weight of the composition.

Detersive Surfactants

The surfactants utilized to formulate the 2-in-1 shampoos of the disclosed technology can be selected from anionic surfactants, cationic surfactants, amphoteric surfactants, nonionic surfactants, and mixtures thereof.

Non-limiting examples of anionic surfactants are disclosed in McCutcheon's Detergents and Emulsifiers, North American Edition, 1998, published by Allured Publishing Corporation; and McCutcheon's, Functional Materials, North American Edition (1992); both of which are incorporated by reference herein in their entirety. The anionic surfactant can be any of the anionic surfactants known or previously used in the art of aqueous surfactant compositions. Suitable anionic surfactants include but are not limited to alkyl sulfates, alkyl ether sulfates, alkyl sulphonates, alkaryl sulfonates, $\alpha$-olefin-sulphonates, alkylamide sulphonates, alkarylpolyether sulphates, alkylamidoether sulphates, alkyl monoglyceryl ether sulfates, alkyl monoglyceride sulfates, alkyl monoglyceride sulfonates, alkyl succinates, alkyl sulfosuccinates, alkyl sulfosuccinamates, alkyl ether sulphosuccinates, alkyl amidosulfosuccinates; alkyl sulphoacetates, alkyl phosphates, alkyl ether phosphates, alkyl ether carboxylates, alkyl amidoethercarboxylates, N-alkylamino acids, N-acyl amino acids, alkyl peptides, N-acyl taurates, alkyl isethionates, carboxylate salts wherein the acyl group is derived from fatty acids; and the alkali metal, alkaline earth metal, ammonium, amine, and triethanolamine salts thereof.

In one aspect, the cation moiety of the forgoing salts is selected from sodium, potassium, magnesium, ammonium, mono-, di- and triethanolamine salts, and mono-, di-, and tri-isopropylamine salts. The alkyl and acyl groups of the foregoing surfactants contain from about 6 to about 24 carbon atoms in one aspect, from 8 to 22 carbon atoms in another aspect and from about 12 to 18 carbon atoms in a further aspect and can be saturated or unsaturated. The aryl groups in the surfactants are selected from phenyl or benzyl. The ether containing surfactants set forth above can contain from 1 to 10 ethylene oxide and/or propylene oxide units per surfactant molecule in one aspect, and from 1 to 3 ethylene oxide units per surfactant molecule in another aspect.

Examples of suitable anionic surfactants include but are not limited to the sodium, potassium, lithium, magnesium, and ammonium salts of laureth sulfate, trideceth sulfate, myreth sulfate, $C_{12}$-$C_{13}$ pareth sulfate, $C_{12}$-$C_{14}$ pareth sulfate, and $C_{12}$-$C_{15}$ pareth sulfate, ethoxylated with 1, 2, 3, 4 or 5 moles of ethylene oxide; sodium, potassium, lithium,

magnesium, ammonium, and triethanolamine lauryl sulfate, coco sulfate, tridecyl sulfate, myrstyl sulfate, cetyl sulfate, cetearyl sulfate, stearyl sulfate, oleyl sulfate, and tallow sulfate, disodium lauryl sulfosuccinate, disodium laureth sulfosuccinate, sodium cocoyl isethionate, sodium $C_{12}$-$C_{14}$ olefin sulfonate, sodium laureth-6 carboxylate, sodium methyl cocoyl taurate, sodium cocoyl glycinate, sodium myristyl sarcocinate, sodium dodecylbenzene sulfonate, sodium cocoyl sarcosinate, sodium cocoyl glutamate, potassium myristoyl glutamate, triethanolamine monolauryl phosphate, and fatty acid soaps, including the sodium, potassium, ammonium, and triethanolamine salts of a saturated and unsaturated fatty acids containing from about 8 to about 22 carbon atoms.

The cationic surfactants can be any of the cationic surfactants known or previously used in the art of aqueous surfactant compositions. Useful cationic surfactants can be one or more of those described, for example, in McCutcheon's Detergents and Emulsifiers, North American Edition, 1998, supra, and Kirk-Othmer, Encyclopedia of Chemical Technology, 4th Ed., Vol. 23, pp. 478-541, the contents of which are herein incorporated by reference. Suitable classes of cationic surfactants include but are not limited to alkyl amines, alkyl imidazolines, ethoxylated amines, quaternary compounds, and quaternized esters. In addition, alkyl amine oxides can function as a cationic surfactant at a low pH.

Alkylamine surfactants can be salts of primary, secondary and tertiary fatty $C_{12}$-$C_{22}$ alkylamines, substituted or unsubstituted, and substances sometimes referred to as "amidoamines". Non-limiting examples of alkylamines and salts thereof include dimethyl cocamine, dimethyl palmitamine, dioctylamine, dimethyl stearamine, dimethyl soyamine, soyamine, myristyl amine, tridecyl amine, ethyl stearylamine, N-tallowpropane diamine, ethoxylated stearylamine, dihydroxy ethyl stearylamine, arachidylbehenylamine, dimethyl lauramine, stearylamine hydrochloride, soyamine chloride, stearylamine formate, N-tallowpropane diamine dichloride, and amodimethicone.

Non-limiting examples of amidoamines and salts thereof include stearamido propyl dimethyl amine, stearamidopropyl dimethylamine citrate, palmitamidopropyl diethylamine, and cocamidopropyl dimethylamine lactate.

Non-limiting examples of alkyl imidazoline surfactants include alkyl hydroxyethyl imidazoline, such as stearyl hydroxyethyl imidazoline, coco hydroxyethyl imidazoline, ethyl hydroxymethyl oleyl oxazoline, and the like.

Non-limiting examples of ethyoxylated amines include PEG-cocopolyamine, PEG-15 tallow amine, quaternium-52, and the like.

Among the quaternary ammonium compounds useful as cationic surfactants, some correspond to the general formula: $(R^{20}R^{21}R^{22}R^{23}N^+)\ E^-$, wherein $R^{20}$, $R^{21}$, $R^{22}$, and $R^{23}$ are independently selected from an aliphatic group having from 1 to about 22 carbon atoms, or an aromatic, alkoxy, polyoxyalkylene, alkylamido, hydroxyalkyl, aryl or alkylaryl group having 1 to about 22 carbon atoms in the alkyl chain; and E- is a salt-forming anion such as those selected from halogen, (e.g., chloride, bromide), acetate, citrate, lactate, glycolate, phosphate, nitrate, sulfate, and alkylsulfate. The aliphatic groups can contain, in addition to carbon and hydrogen atoms, ether linkages, ester linkages, and other groups such as amino groups. The longer chain aliphatic groups, e.g., those of about 12 carbons, or higher, can be saturated or unsaturated. In one aspect, the aryl groups are selected from phenyl and benzyl.

Exemplary quaternary ammonium surfactants include, but are not limited to cetyl trimethylammonium chloride, cetylpyridinium chloride, dicetyl dimethyl ammonium chloride, dihexadecyl dimethyl ammonium chloride, stearyl dimethyl benzyl ammonium chloride, dioctadecyl dimethyl ammonium chloride, dieicosyl dimethyl ammonium chloride, didocosyl dimethyl ammonium chloride, dihexadecyl dimethyl ammonium chloride, dihexadecyl dimethyl ammonium acetate, behenyl trimethyl ammonium chloride, benzalkonium chloride, benzethonium chloride, and di(coconutalkyl) dimethyl ammonium chloride, ditallowdimethyl ammonium chloride, di(hydrogenated tallow) dimethyl ammonium chloride, di(hydrogenated tallow) dimethyl ammonium acetate, ditallowdimethyl ammonium methyl sulfate, ditallow dipropyl ammonium phosphate, and ditallow dimethyl ammonium nitrate.

At low pH, amine oxides can protonate and behave similarly to N-alkyl amines. Examples include, but are not limited to, dimethyl- dodecylamine oxide, oleyldi(2-hydroxyethyl) amine oxide, dimethyltetradecylamine oxide, di(2-hydroxyethyl)-tetradecylamine oxide, dimethylhexadecylamine oxide, behenamine oxide, cocamine oxide, decyltetradecylamine oxide, dihydroxyethyl $C_{12}$-$C_{15}$ alkoxypropylamine oxide, dihydroxyethyl cocamine oxide, dihydroxyethyl lauramine oxide, dihydroxyethyl stearamine oxide, dihydroxyethyl tallowamine oxide, hydrogenated palm kernel amine oxide, hydrogenated tallowamine oxide, hydroxyethyl hydroxypropyl $C_{12}$-$C_{15}$ alkoxypropylamine oxide, lauramine oxide, myristamine oxide, cetylamine oxide, oleamidopropylamine oxide, oleamine oxide, palmitamine oxide, PEG-3 lauramine oxide, dimethyl lauramine oxide, potassium trisphosphonomethylamine oxide, soyamidopropylamine oxide, cocamidopropylamine oxide, stearamine oxide, tallowamine oxide, and mixtures thereof.

The term "amphoteric surfactant" as used herein, is also intended to encompass zwitterionic surfactants, which are well known to formulators skilled in the art as a subset of amphoteric surfactants. Nonlimiting examples of amphoteric surfactants are disclosed *McCutcheon's Detergents and Emulsifiers,* North American Edition, supra, and McCutcheon's, *Functional Materials,* North American Edition, supra; both of which are incorporated by reference herein in their entirety. Suitable examples include but are not limited to amino acids (e.g., N-alkyl amino acids and N-acyl amino acids), betaines, sultaines, and alkyl amphocarboxylates.

Amino acid based surfactants suitable in the practice of the present invention include surfactants represented by the formula:

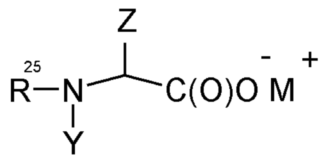

wherein $R^{25}$ represents a saturated or unsaturated hydrocarbon group having 10 to 22 carbon atoms or an acyl group containing a saturated or unsaturated hydrocarbon group having 9 to 22 carbon atoms, Y is hydrogen or methyl, Z is selected from hydrogen, $-CH_3$, $-CH(CH_3)_2$, $-CH_2CH(CH_3)_2$, $-CH(CH_3)CH_2CH_3$, - $CH_2C_6H_5$, $-CH_2C_6H_4OH$, $-CH_2OH$, $-CH(OH)CH_3$, $-(CH_2)_4NH_2$, $-(CH_2)_3NHC(NH)NH_2$, $-CH_2C(O)O^-M^+$, $-(CH_2)_2C(O)O^-M^+$. M is a salt forming cation. In one aspect, $R^{25}$ represents a radical selected from a linear or branched $C_{10}$ to $C_{22}$ alkyl group, a linear or branched $C_{10}$ to $C_{22}$ alkenyl group, an acyl group represented by $R^{26}C(O)$-, wherein $R^{26}$ is selected from a linear or branched $C_9$ to $C_{22}$ alkyl group, a linear or branched $C_9$ to $C_{22}$ alkenyl group. In one aspect, $M^+$ is a cation selected from sodium, potassium, ammonium, and triethanolamine (TEA).

The amino acid surfactants can be derived from the alkylation and acylation of $\alpha$-amino acids such as, for example, alanine, arginine, aspartic acid, glutamic acid, glycine, isoleucine, leucine, lysine, phenylalanine, serine, tyrosine, and valine. Representative N-acyl amino acid surfactants are, but not limited to the mono- and di- carboxylate salts (e.g., sodium, potassium, ammonium and TEA) of N-acylated glutamic acid, for example, sodium cocoyl glutamate, sodium lauroyl glutamate, sodium myristoyl glutamate, sodium palmitoyl glutamate, sodium stearoyl glutamate, disodium cocoyl glutamate, disodium stearoyl glutamate, potassium cocoyl glutamate, potassium lauroyl glutamate, and potassium myristoyl glutamate; the carboxylate salts (e.g., sodium, potassium, ammonium and TEA) of N-acylated alanine, for example, sodium cocoyl alaninate, and TEA lauroyl alaninate; the carboxylate salts (e.g., sodium, potassium, ammonium and TEA) of N-acylated glycine, for example, sodium cocoyl glycinate, and potassium cocoyl glycinate; the carboxylate salts (e.g., sodium, potassium, ammonium and TEA) of N-acylated sarcosine, for example, sodium lauroyl sarcosinate, sodium cocoyl sarcosinate, sodium myristoyl sarcosinate, sodium oleoyl sarcosinate, and ammonium lauroyl sarcosinate; and mixtures of the foregoing surfactants.

The betaines and sultaines useful in the present invention are selected from alkyl betaines, alkylamino betaines, and alkylamido betaines, as well as the corresponding sulfobetaines (sultaines) represented by the formulas:

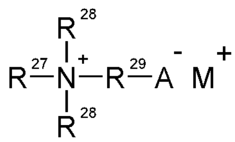

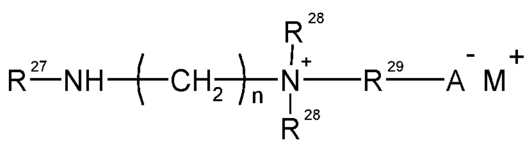

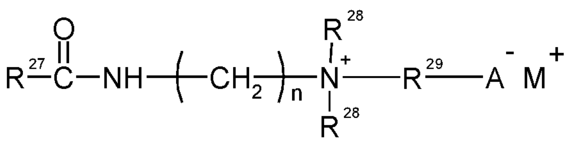

wherein $R^{27}$ is a $C_7$-$C_{22}$ alkyl or alkenyl group, each $R^{28}$ independently is a $C_1$-$C_4$ alkyl group, $R^{29}$ is a $C_1$-$C_5$ alkylene group or a hydroxy substituted $C_1$-$C_5$ alkylene group, n is an integer from 2 to 6, A is a carboxylate or sulfonate group, and M is a salt forming cation. In one aspect, $R^{27}$ is a $C_{11}$-$C_{18}$ alkyl group or a $C_{11}$-$C_{18}$ alkenyl group. In one aspect, $R^{28}$ is methyl. In one aspect, $R^{29}$ is methylene, ethylene or hydroxy propylene. In one aspect, n is 3. In a further aspect, M is selected from sodium, potassium, magnesium, ammonium, and mono-, di- and triethanolamine cations.

Examples of suitable betaines include, but are not limited to, lauryl betaine, coco betaine, oleyl betaine, cocohexadecyl dimethylbetaine, lauryl amidopropyl betaine, cocoamidopropyl betaine (CAPB), and cocamidopropyl hydroxysultaine.

The alkylamphocarboxylates such as the alkylamphoacetates and alkylamphopropionates (mono- and disubstituted carboxylates) can be represented by the formula:

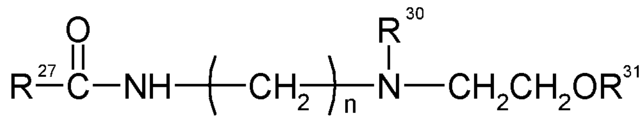

wherein $R^{27}$ is a $C_7$-$C_{22}$ alkyl or alkenyl group, $R^{30}$ is $-CH_2C(O)O^- M^+$, $- CH_2CH_2C(O)O^- M^+$, or $-CH_2CH(OH)CH_2SO_3^- M^+$, $R^{31}$ is hydrogen or $-CH_2C(O)O^- M^+$, and M is a cation selected from sodium, potassium, magnesium, ammonium, and mono-, di- and triethanolamine.

Exemplary alkylamphocarboxylates include, but are not limited to, sodium cocoamphoacetate, sodium lauroamphoacetate, sodium capryloamphoacetate, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, and disodium capryloamphodipropionate.

Non-limiting examples of nonionic surfactants are disclosed in McCutcheon's Detergents and Emulsifiers, North American Edition, 1998, supra; and McCutcheon's, *Functional Materials,* North American, supra; both of which are incorporated by reference herein in their entirety. Additional Examples of nonionic surfactants are described in U.S. Patent No. 4,285,841, to Barrat et al., and U.S. Patent. No. 4,284,532, to Leikhim et al., both of which are incorporated by reference herein in their entirety. Nonionic surfactants typically have a hydrophobic portion, such as a long chain alkyl group or an alkylated aryl group, and a hydrophilic portion containing various degrees of ethoxylation and/or propoxylation (e.g., 1 to about 50) ethoxy and/or propoxy moieties. Examples of some classes of nonionic surfactants that can be used include, but are not limited to, ethoxylated alkylphenols, ethoxylated and propoxylated fatty alcohols, polyethylene glycol ethers of methyl glucose, polyethylene glycol ethers of sorbitol, ethylene oxide-propylene oxide block copolymers, ethoxylated esters of fatty acids, condensation products of ethylene oxide with long chain amines or amides, condensation products of ethylene oxide with alcohols, and mixtures thereof.

Suitable nonionic surfactants include, for example, alkyl polysaccharides, alcohol ethoxylates, block copolymers, castor oil ethoxylates, ceto/oleyl alcohol ethoxylates, cetearyl alcohol ethoxylates, decyl alcohol ethoxylates, dinonyl phenol ethoxylates, dodecyl phenol ethoxylates, end-capped ethoxylates, ether amine derivatives, ethoxylated alkanolamides, ethylene glycol esters, fatty acid alkanolamides, fatty alcohol alkoxylates, lauryl alcohol ethoxylates, mono-branched alcohol ethoxylates, nonyl phenol ethoxylates, octyl phenol ethoxylates, oleyl amine ethoxylates, random copolymer alkoxylates, sorbitan ester ethoxylates, stearic acid ethoxylates, stearyl amine ethoxylates, tallow oil fatty acid ethoxylates, tallow amine ethoxylates, tridecanol ethoxylates, acetylenic diols, polyoxyethylene sorbitols, and mixtures thereof. Various specific examples of suitable nonionic surfactants include, but are not limited to, methyl gluceth-10, PEG-20 methyl glucose distearate, PEG-20 methyl glucose sesquistearate, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene- 10 oleyl ether, polyoxyethylene-20 oleyl ether, an ethoxylated nonylphenol, ethoxylated octylphenol, ethoxylated dodecylphenol, or ethoxylated fatty ($C_6$-$C_{22}$) alcohol, including 3 to 20 ethylene oxide moieties, polyoxyethylene-20 isohexadecyl ether, polyoxyethylene-23 glycerol laurate, polyoxyethylene-20 glyceryl stearate, PPG-10 methyl glucose ether, PPG-20 methyl glucose ether, polyoxyethylene-20 sorbitan monoesters, polyoxyethylene-80 castor oil, polyoxyethylene-15 tridecyl ether, polyoxyethylene-6 tridecyl ether, laureth-2, laureth-3, laureth-4, PEG-3 castor oil, PEG 600 dioleate, PEG 400 dioleate, poloxamers such as poloxamer 188, polysorbate 21, polysorbate 40, polysorbate 60, polysorbate 61, polysorbate 65, polysorbate 80, polysorbate 81, polysorbate 85, sorbitan caprylate, sorbitan cocoate, sorbitan diisostearate, sorbitan dioleate, sorbitan distearate, sorbitan fatty acid ester, sorbitan isostearate, sorbitan laurate, sorbitan oleate, sorbitan palmitate, sorbitan sesquiisostearate, sorbitan sesquioleate, sorbitan sesquistearate, sorbitan stearate, sorbitan triisostearate, sorbitan trioleate, sorbitan tristearate, sorbitan undecylenate, or mixtures thereof.

Alkyl glycoside nonionic surfactants can also be employed and are generally prepared by reacting a monosaccharide, or a compound hydrolyzable to a monosaccharide, with an alcohol such as a fatty alcohol in an acid medium. For example, U.S. Patent Nos. 5,527,892 and 5,770,543 describe alkyl glycosides and/or methods for their preparation. Suitable examples are commercially available under the names of Glucopon™ 220, 225, 425, 600 and 625, PLANTACARE®, and PLANTAPON®, all of which are available from Cognis Corporation of Ambler, Pennsylvania.

In another aspect, nonionic surfactants include, but are not limited to, alkoxylated methyl glucosides such as, for example, methyl gluceth-10, methyl gluceth-20, PPG-10 methyl glucose ether, and PPG-20 methyl glucose ether, available from Lubrizol Advanced Materials, Inc., under the trade names, Glucam® E10, Glucam® E20, Glucam® P10, and Glucam® P20, respectively; and hydrophobically modified alkoxylated methyl glucosides, such as PEG 120 methyl glucose dioleate, PEG-120 methyl glucose trioleate, and PEG-20 methyl glucose sesquistearate, available from Lubrizol Advanced Materials, Inc., under the trade names, Glucamate® DOE-120, Glucamate™ LT, and Glucamate™ SSE-20, respectively, are also suitable. Other exemplary hydrophobically modified alkoxylated methyl glucosides are disclosed in U.S. Patent Nos. 6,573,375 and 6,727,357, the disclosures of which are hereby incorporated by reference in their entirety.

Other useful nonionic surfactants include water soluble silicones such as PEG-10 Dimethicone, PEG-12 Dimethicone, PEG-14 Dimethicone, PEG-17 Dimethicone, PPG-12 Dimethicone, PPG-17 Dimethicone and derivatized/functionalized forms thereof such as Bis-PEG/PPG-20/20 Dimethicone Bis-PEG/PPG-16/16 PEG/PPG-16/16 Dimethicone, PEG/PPG-14/4 Dimethicone, PEG/PPG-20/20 Dimethicone, PEG/PPG-20/23 Dimethicone, and Perfluorononylethyl Carboxydecyl PEG-10 Dimethicone.

The amount of the at least one surfactant (active weight basis) utilized in formulating the 2-in-one shampoos of the disclosed technology ranges from about 1 to about 50 wt.%, or from about 5 to about 40 wt.%, or from about 10 to about 35 wt.%, or from about 15 to about 30 wt.%, based on the total weight of the composition. In another aspect, the amount of the at least one surfactant utilized in the formulation ranges from about 3 to about 25 wt.%. In still another aspect, the amount of the at least one surfactant employed ranges from about 5 to about 22 wt.%. In a further aspect, the amount of the at least one surfactant utilized ranges from about 6 to about 20 wt.%. In still a further aspect, the amount of at least one surfactant is about 10, 12, 14, 16, and 18 wt.% based on the total weight yield of the composition.

Water

Water is also an ingredient in the compositions according to embodiments of the disclosed technology. In one aspect, the liquid cleansing compositions described herein are in the form of non-emulsion liquids in which water is the principal carrier/diluent/carrier. Considering the desired amounts (wt.%) of the other active components utilized to formulate the compositions of the disclosed technology, the quantity of water employed in the compositions will always correspond to a weight percentage needed to bring the total weight of the composition to 100 (i.e., quantity sufficient (q.s.) to 100). In another aspect, the amount of water can range from about 25 to about 89.5 wt.%, in a further aspect from about 35 to about 85 wt.%, in a still further aspect from about 40 to about 80 wt.%, in an additional aspect from about 40 to about 75 wt.%, in a still additional aspect from about 50 to about 70 wt.%, and a further additional aspect from about 55 to about 65 wt.%, based on the total weight of the composition.

pH Adjusting Agents

In one aspect, the pH of the cleansing compositions of the disclosed technology can range from about 4 to about 10.5, or about 5 to about 8, or about 5.5 to about 6.5. A sufficient amount of a pH adjusting agent (base and/or acid) can be added to the compositions of the disclosed technology to attain the desired pH.

Many types of alkaline (basic) pH adjusting agents can be used, including inorganic and organic bases, and combinations thereof. Examples of inorganic bases include but are not limited to the ammonium and alkali metal hydroxides (especially sodium and potassium), and alkali metal salts of inorganic acids, such as sodium borate (borax), sodium phosphate, sodium pyrophosphate, and the like; and mixtures thereof. Examples of organic bases include, but are not limited to, triethanolamine (TEA), diisopropanolamine, triisopropanolamine, aminomethyl propanol, dodecylamine, cocamine, oleamine, morpholine, triamylamine, triethylamine, tetrakis(hydroxypropyl)ethylenediamine, L-arginine, aminomethyl propanol, tromethamine (2-amino 2-hydroxymethyl-1,3-propanediol), and PEG-15 cocamine.

In one aspect, the acidic pH adjusting agents are selected from an organic acid, such as citric acid, acetic acid, alpha-hydroxy acids, beta-hydroxy acids, salicylic acid, lactic acid, fumaric acid, glutamic acid, glycolic acid, tartaric acid, natural fruit acids, or combinations thereof. In addition, inorganic acids, for example, hydrochloric acid, nitric acid, sulfuric acid, sodium bisulfate, sulfamic acid, phosphoric acid, and combinations thereof can be utilized. Mixtures of organic acids and inorganic acids are also contemplated.

In one aspect, a basic pH adjusting agent is added to the composition in an amount sufficient to attain the desired target pH for the composition. In one aspect, an acidic pH adjusting agent is added to the composition in an amount sufficient to attain the desired target pH for the composition. In one aspect, a basic pH adjusting agent is added to the composition, followed by the addition of an acidic pH adjusting agent to attain the desired target pH for the composition.

Optional Components

The compositions of the disclosed technology can contain a variety of other conventional optional components suitable for rendering the cleansing compositions more desirable. Such optional components are well known to those skilled in the art of formulating personal care cleansing compositions and include, but not limited to, one or more preservatives, one or more thickening agents, one or more viscosity adjusting agents, one or more skin conditioners, one or more antibacterial agents, one or more fragrances, one or more colorants, and one or more insoluble materials.

Suitable preservatives and antimicrobial agents, if present, include polymethoxy bicyclic oxazolidine, methyl paraben, propyl paraben, ethyl paraben, butyl paraben, benzoic acid and the salts of benzoic acid, e.g., sodium benzoate, benzyltriazole, DMDM hydantoin (also known as 1,3-dimethyl-5,5-dimethyl hydantoin), imidazolidinyl urea, phenoxyethanol, phenoxyethylparaben, methylisothiazolinone, methylchloroisothiazolinone, benzoisothiazolinone, triclosan, sorbic acid, salicylic acid salts, and the like, and mixtures thereof. Preservatives typically comprise about 0.01 wt. % to about 1.5 wt. % of the total wt. of the personal care compositions of the present technology.

Suitable auxiliary thickening agents may be any natural and/or synthetic agent (or combination thereof) to obtain enhanced thickening properties. The person skilled in the art will readily select a proper thickening agent(s) and amounts(s) thereof to obtain the desired rheology. Non-limiting examples of natural thickening agents are tree & shrub exudates (karaya gum, tragacanth gum, gum Arabic, gum ghatti), seed extracts (guar gum, cassia gum, locust been gum, tamarind seed), seaweed extracts (carrageenan, alginates, agar), fruit extracts (pectins, waxes), grains & roots (corn starch, potato starch, etc), microbial polysaccharides (Xanthan gum, dextran), modified natural products (cellulose derivatives such as hydropropyl cellulose, methylcellulose, hydroxypropyl methylcellulose, cellulose gum, etc.); and hydrophobically modified ethoxylated methyl glucosides, such as PEG 120 methyl glucose dioleate, PEG-120 methyl glucose trioleate, and PEG-20 methyl glucose sesquistearate, available from Lubrizol Advanced Materials, Inc., under the trade names, Glucamate™ DOE-120, Glucamate™ LT and VLT, and Glucamate™ SSE-20, respectively, are also suitable as thickening agents. Non-limiting examples of synthetic thickening agents include the polyethylene glycols (PEG) having 5 to 200 glycol units, such as, for example, those available under the INCI names PEG- 6, PEG-8, PEG-12, PEG-20, PEG-30, PEG-32, PEG-75, PEG-90, PEG-100 and PEG-200; acrylic/methacrylic acid homopolymers and copolymers, such as, for example, those sold under the trade names Carbopol® 934, Carbopol 940, Carbopol 941, Carbopol 980, Carbopol 981, Carbopol 2984, Carbopol 5984, Carbopol ETD 2050, Carbopol Ultrez 10, Carbopol Ultrez 30 (INCI name: Carbomer); Carbopol 1342, Carbopol 1382, Carbopol ETD 2020, Carbopol Ultrez 20, Carbopol Ultrez 21, Pemulen™ TR-1 and Pemulen TR-2 (INCI name: Acrylates/$C_{10}$-$C_{30}$ Alkyl Acrylate Crosspolymer); Carbopol Aqua SF-1 (INCI name: Acrylates Copolymer); manufactured and sold by Lubrizol Advanced Materials, Inc., Cleveland, OH; acrylamide homopolymers and copolymers; polymers prepared from 2-acrylamido-2-methylpropanesulfonic acid (AMPS® monomer).

Another class of synthetic thickeners suitable for use includes the hydrophobically modified alkali-swellable emulsion polymers, commonly referred to as (HASE) polymers. Typical HASE polymers are free radical addition polymers polymerized from pH sensitive or hydrophilic monomers (e.g., acrylic acid and/or methacrylic acid, 2-acrylamido-2-methylpropane sulfonic acid), hydrophobic monomers (e.g., $C_1$-$C_{30}$ alkyl esters of acrylic acid and/or methacrylic acid, acrylonitrile, styrene), an "associative monomer", and an optional crosslinking monomer. The associative monomer comprises an ethylenically unsaturated polymerizable end group, a non-ionic hydrophilic midsection that is terminated by a hydrophobic end group. The non-ionic hydrophilic midsection comprises a polyoxyalkylene group, e.g., polyethylene oxide, polypropylene oxide, or mixtures of polyethylene oxide/polypropylene oxide segments. The terminal hydrophobic end group is typically a $C_8$-$C_{40}$ aliphatic moiety. Exemplary aliphatic moieties are selected from linear and branched alkyl substituents, linear and branched alkenyl substituents, carbocyclic substituents, aryl substituents, aralkyl substituents, arylalkyl substituents, and alkylaryl substituents. In one aspect, associative monomers can be prepared by the condensation (e.g., esterification or etherification) of a polyethoxylated and/or polypropoxylated aliphatic alcohol (typically containing a branched or unbranched $C_8$-$C_{40}$ aliphatic moiety) with an ethylenically unsaturated monomer containing a carboxylic acid group (e.g., acrylic acid, methacrylic acid), an unsaturated cyclic anhydride monomer (e.g., maleic anhydride, itaconic anhydride, citraconic anhydride), a monoethylenically unsaturated monoisocyanate (e.g., $\alpha$,$\alpha$-dimethyl-m-isopropenyl benzyl isocyanate) or an ethylenically unsaturated monomer containing a hydroxyl group (e.g., vinyl alcohol, allyl alcohol). Polyethoxylated and/or polypropoxylated aliphatic alcohols are ethylene oxide and/or propylene oxide adducts of a monoalcohol containing the $C_8$-$C_{40}$ aliphatic moiety. Non-limiting examples of alcohols containing a $C_8$-$C_{40}$ aliphatic moiety are capryl alcohol, iso-octyl alcohol (2-ethyl hexanol), pelargonic alcohol (1-nonanol), decyl alcohol, lauryl alcohol, myristyl alcohol, cetyl alcohol, cetyl alcohol, cetearyl alcohol (mixture of $C_{16}$-$C_{18}$ monoalcohols), stearyl alcohol, isostearyl alcohol, elaidyl alcohol, oleyl alcohol, arachidyl alcohol, behenyl alcohol, lignoceryl alcohol, ceryl alcohol, montanyl alcohol, melissyl, lacceryl alcohol, geddyl alcohol, and $C_2$-$C_{20}$ alkyl substituted phenols (e.g., nonyl phenol), and the like.

Exemplary HASE polymers are disclosed in U.S. Patent Nos. 3,657,175; 4,384,096; 4,464,524; 4,801,671; and 5,292,843, which are herein incorporated by reference. In addition, an extensive review of HASE polymers is found in Gregory D. Shay, Chapter 25, "Alkali-Swellable and Alkali-Soluble Thickener Technology A Review", Polymers in Aqueous Media - Performance Through Association, Advances in Chemistry Series 223, J. Edward Glass (ed.), ACS, pp. 457-494, Division Polymeric Materials, Washington, DC (1989), the relevant disclosures of which are incorporated herein by reference. Commercially available HASE polymers are sold under the trade names, Aculyn® 22 (INCI Name: Acrylates/Steareth-20 Methacrylate Copolymer), Aculyn® 44 (INCI Name: PEG-150/Decyl Alcohol/SMDI Copolymer), Aculyn 46® (INCI Name: PEG-150/Stearyl Alcohol/SMDI Copolymer), and Aculyn® 88 (INCI Name: Acrylates/Steareth-20 Methacrylate Crosspolymer) from Rohm & Haas, and Novethix™ L-10 (INCI Name: Acrylates/Beheneth-25 Methacrylate Copolymer) from Lubrizol Advanced Materials, Inc. Other thickeners are commercially available under the INCI designations Ammonium Acryloyldimethyltaurate/VP Copolymer, Ammonium Acryloyl Dimethyltaurate/Carboxyethyl Acrylate Crosspolymer, and Ammonium Acryloyldimethyltaurate/Beheneth-25 Methacrylate Crosspolymer.

If utilized, the thickeners can comprise from about 0.01 wt. % to about 5 wt. % of the total weight of the personal care composition. in another aspect the amount ranges from about 0.1 wt. % to about 3 wt. %, and in a further aspect from about 0.1 wt. % to about 2.0 wt. % of the total weight of the personal care composition.

Viscosity adjusting agents are used in cosmetics to enhance the fluidity of products without a significant lowering of the concentration of the active constituents. Suitable viscosity adjusting agents if present include organic and inorganic compounds, and combinations thereof. Examples of organic compounds include ethanol, isopropyl alcohol, sorbitol, propylene glycol, diethylene glycol, triethylene glycol, dimethyl ether, butylene glycol, and the like, and mixtures thereof. Examples of inorganic compounds include sodium chloride, sodium sulfate, potassium chloride, potassium nitrate, and mixtures thereof. If utilized the viscosity adjusting agents typically comprise from about 0.1 wt. % to about 20 wt.% in one aspect, and from about 1 wt.% to about 5 wt.% of the total weight of the composition of the disclosed technology.

Examples of suitable antibacterial agents which can be used herein include, but are not limited to, 2-hydroxy-4,2',4'-trichlorodiphenylether (TCS), 2,6-dimethyl-4-hydroxychlorobenzene (PCMX),3,4,4'-trichlorocarbanilide (TCC), 3-trifluoromethyl-4,4'-dichlorocarbanilide (TFC), 2,2'-dihydroxy-3,3',5,5',6,6'-hexachlorodiphenylmethane, 2,2'-dihydroxy-3,3',5,5'-tetrachlorodiphenylmethane, 2,2'-dihydroxy-3,3',dibromo-5,5'-dichlorodiphenylmethane, 2-hydroxy4,4'-dichlorodiphenylether, 2-hydroxy-3,5',4-tribromodiphenylether, 1-hydroxyl-4-methyl-6-(2,4,4-trimethylpentyl)-2(1H)-pyridinone (Octopirox), salts of 2-pyridinethiol-1-oxide, salicylic acid, and other organic acids. Other suitable antibacterial agents are described in U.S. Patent. Nos. 3,835,057; 4,714,653; and 6,488,943. The disclosed composition can include from about 0.001 wt.% to about 2 wt.% in one aspect, from about 0.01 wt.% to about 1.5 wt.% in another aspect, and from about 0.1 wt.% to about 1 wt.% in a further aspect of the antibacterial agent(s), based on the total weight of the composition.

The fragrance substances that can be used in the compositions of the disclosed technology include natural and synthetic fragrances, perfumes, scents, and essences and any other substances and mixtures which emit a fragrance. As the natural fragrances, there are those of vegetable origin, such as oil extracts from flowers (e.g., lily, lavender, rose, jasmine, neroli, ylang-ylang), stems and leaves (geranium, patchouli, petitgrain, peppermint), fruits (aniseed, coriander, fennel, needle juniper), fruit skin (bergamot, lemon, orange, mace), roots angelica, celery, cardamom, costus, iris, sweet flag), woods (pine tree, sandalwood, guaiacum wood, cedar, rosewood, cinnamon), herbs and grasses (tarragon, lemongrass, sage, thyme), needles and twigs (spruce, pine, European red pine, stone pine), and resins and balsam (galbanum, elemi, benzoin, myrrh, frankincense, opopanax), and those of animal origin, such as musk, civet, castoreum, ambergris, or the like, and mixtures thereof.

Examples of synthetic fragrances and perfumes are the aromatic esters, ethers, aldehydes, ketones, alcohols, and hydrocarbons including, but are not limited to, benzyl acetate, phenoxyethyl isobutylate, p-tert-butylcyclohexyl acetate, linalyl acetate, dimethylbenzylcarbinyl acetate, phenylethyl acetate, linalyl benzoate, benzyl formate, ethylmethylphenyl glycinate, allylcyclohexyl propionate, styralyl propionate, and benzyl salicylate; benzylethyl ether; straight chain alkanals having 8 to 18 carbon atoms, citral, citronellal, citronellyloxyaldehyde, cyclamen aldehyde, hydroxycitronellal, lilial, and bougeonal; ionone compounds, $\alpha$-isomethyl ionone, and methyl cedryl ketone; anethole, citronellol, eugenol, isoeugenol, geraniol, lavandulol, nerolidol, linalool, phenylethyl alcohol, and terpineol, alpha-pinene, terpenes (e.g., limonene), and balsams, and mixtures thereof.

The amount of fragrance material(s) that can be utilized will depend on the preference of the skilled formulator. In one aspect, the amount of fragrance material can range from about 0.05 wt.% to about 3 wt. %, in another aspect from about 0.1 wt.% to about 1.5 wt.%, in still another aspect from about 0.3 wt.% to about 1 wt.%, and in a further aspect from about 0.5 wt.% to 0.75 wt.%, based on the weight of the total composition.

Colorants include water soluble dyes such as copper sulfate, iron sulfate, water-soluble sulfopolyesters, rhodamines, natural dyes, for instance carotene and beetroot juice, methylene blue, caramel, the disodium salt of tartrazine and the disodium salt of fuschin, and mixtures thereof. The amount of colorant(s) employed in the composition will depend on the aesthetic preference of the skilled formulator.

Insoluble materials include materials that impart pearlescent and other aesthetic visual, sensory and/or beneficial effects to the composition. Some formulations are opacified by deliberately incorporating pearlescent materials therein to achieve a cosmetically attractive pearl-like appearance, known as pearlescence. A detailed discussion of the effect is found in the article "Opacifiers and pearling agents in shampoos" by Hunting, Cosmetic and Toiletries, Vol. 96, pages 65 to 78 (July 1981), incorporated herein by reference.

The pearlescent material includes titanium dioxide coated mica, iron oxide coated mica, ethylene glycol monostearate, ethylene glycol distearate, polyethylene glycol distearate, bismuth oxychloride coated mica, myristyl myristate, guanine, glitter (polyester or metallic), and mixtures thereof. Other pearlescent materials can be found in U.S. Patent No. 4,654,207 and U.S. Patent No. 5,019,376, herein incorporated by reference.

The amount of the pearlescent material can generally be used in amounts of from about 0.05% to about 10% and desirably from about 0.15% to about 3% by wt. based upon the total wt. of the composition.

In addition to the above generally insoluble compounds, numerous other optional substantially insoluble compounds which require stabilization can be utilized in the composition. Examples of such other insoluble compounds

include titanium dioxide; pumice; calcium carbonate, talc, potato starch, tapioca starch, jojoba beads, polyethylene beads, walnut shells, loofah, apricot seeds; almond meal, corn meal, paraffin, oat bran/oat hulls, gelatin beads, alginate beads, stainless steel fibers, iron oxide pigments, air bubbles, mica coated iron oxides, kaolin clay, salicylic acid, zinc oxide, zeolite, styrofoam beads, phosphates, silica, and the like. Other generally insoluble compounds include teatree powder, microsponges, Confetti (a trademark of united guardian company), talc, beeswax, and the like. The amount of the various insoluble compounds requiring stabilization will vary depending upon its purpose, desired end result, and efficacy thereof. Hence amounts can vary widely, but frequently will be within a general range of from about 0.1% to about 20% by wt. based upon the total wt. of the composition.

## EXAMPLES

The disclosed technology is illustrated by the following examples that are merely for the purpose of illustration and are not to be regarded as limiting the scope of the claims or the manner in which it can be practiced. Unless specifically indicated otherwise, parts and percentages are given by weight.

### Example 1

2-in-1 shampoo formulations for a silicone deposition study are listed in Table I. The formulations comprise 11.5% total surfactant level (8.9% Sodium Laureth-1 Sulfate, and 2.6% Cocamidopropyl Betaine), 0.25% cationic guar, 2% silicone emulsion (Dow Corning DC1352) and 1% NaCl with different rheology modifiers were prepared. The rheology modifiers for this study includes FDC (INCI: Cellulose (and) Cellulose Gum (and) Glycerin) designated as Sample A and Sample B supplied by CP Kelco, Betafib™ MCF supplied by Royal Cosun Biobased Products (Betafib is a cellulosic microfiber derived from vegetable raw materials), Carbopol™ 980 polymer (C980) a Carbomer obtained from Lubrizol Advanced Materials, Inc., Carbopol™ Aqua SF-1 (SF-1) a crosslinked emulsion copolymer of (meth)acrylic acid and a $C_1$ to $C_5$ alkyl (meth)acrylate obtained from Lubrizol Advanced Materials, Inc. and Carboxyl Methyl Cellulose (CMC). The control formula does not contain rheology modifier.

Yield Value Measurement

Yield Value, also referred to as Yield Stress, is defined as the initial resistance to flow under stress. It is measured by the Brookfield Yield Value (BYV) Extrapolation Method using a Brookfield viscometer (Model DV2TLV Extra) at ambient room temperature of about 21 to 23°C. The viscometer is used to measure the torque necessary to rotate a spindle through a liquid sample at speeds of 0.5 to 100 rpm. Multiplying the torque reading by the appropriate constant for the spindle and speed gives the apparent viscosity. Yield Value is an extrapolation of measured values to a shear rate of zero. The BYV is calculated by the following equation:

$$\text{BYV, dyn/cm}^2 = (\eta_{\alpha 1} - \eta_{\alpha 2})/100$$

where $\eta_{\alpha 1}$ and $\eta_{\alpha 2}$ = apparent viscosities obtained at two different spindle speeds (0.5 rpm and 1.0 rpm, respectively). These techniques and the usefulness of the yield value measurement are explained in Technical Data Sheet Number 244 (Revision: 1/2002) from Lubrizol Advanced Materials, Inc., herein incorporated by reference.

The addition of rheology modifier increases the yield value of the formula. In Table 1, the addition of FDC to the formula significantly increase the yield value of the formula that helps to improve the stability of the formula. It is same as the other rheology modifiers, such as C980 polymer and SF-1, and a combination of FDC & C980.

Brookfield Viscosity

Viscosity measurements for the compositions are conducted by the Brookfield method employing a rotating spindle Brookfield viscometer, Model DV2TLV Extra, (Ametek Brookfield), at 20 revolutions per minute (rpm), at ambient room temperature of about 20 to 25°C (BV viscosity). Spindle sizes are selected in accordance with the standard operating recommendations from the manufacturer. The artisan of ordinary skill in the art will select a spindle size appropriate for the system to be measured.

The silicone deposition is based on the washing protocol developed by Lubrizol to treat a non-damaged European brown hair two times with the testing shampoo. After air drying the hair, the amount of silicone deposited on the hair tress is measured by Hitachi benchtop SEM equipped with EDS. Each data point is the average of two hair tresses and each sample from each hair tress is measured six to nine different locations. The silicone deposition relative index (RI) is the relative amount of silicone of each sample to the sample treated with the control formula.

As illustrated in Figure 1, formulations containing either C980 or CMC gives similar silicone deposition as the control formula with RI=1. It indicates that the rheology modifier of C980 & CMC has no impact on silicone deposition. The formula containing SF-1 exhibits very low silicone deposition onto the hair or RI=0. It means SF-1 has a significantly negative impact on silicone deposition. The formula containing either Cosun MCF or the combination of Cosun MCF and CMC shows lower silicone deposition than the control formula (RI<1) or has negative impact on silicone deposition. However, the formula with FDC Sample A, FDC Sample B or the combination of FDC and C980 shows a higher silicone deposition relative index, RI > 1. It indicates the FDC supplied by CP Kelco increases silicone deposition from the 2-in-1 shampoo formula with 20% to 40% higher values than the control.

TABLE 1

| Sample Label | Control | Sample A | Sample B | C980 | Sample A/ C980 | Sample B/ C980 | Sample A (w/o C13s) | Sample B (w/o C13s) | Cosun MCF | CMC | Cosun MCF + CMC | SF-1 |
|---|---|---|---|---|---|---|---|---|---|---|---|---|
| DI Water | 25 | 25 | 25 | 25 | 25 | 25 | 25 | 25 | 25 | 25 | 25 | 25 |
| Cationic Guar (Jaguar C13 S)[1] | 0.25 | 0.25 | 0.25 | 0.25 | 0.25 | 0.25 | - | - | 0.25 | 0.25 | 0.25 | 0.25 |
| Sulfochem ES-1K (27%)[2] | 33 | 33 | 33 | 33 | 33 | 33 | 33 | 33 | 33 | 33 | 33 | 33 |
| Chembetaine CAD (35%)[3] | 7.5 | 7.5 | 7.5 | 7.5 | 7.5 | 7.5 | 7.5 | 7.5 | 7.5 | 7.5 | 7.5 | 7.5 |
| Sodium Benzoate | 0.5 | 0.5 | 0.5 | 0.5 | 0.5 | 0.5 | 0.5 | 0.5 | 0.5 | 0.5 | 0.5 | 0.5 |
| Silicone Emulsion DC1352 (60%)[4] | 3.33 | 3.33 | 3.33 | 3.33 | 3.33 | 3.33 | 3.33 | 3.33 | 3.33 | 3.33 | 3.33 | 3.33 |
| Sample A (~1%)[7] | | 5 | | | 2.5 | | 5 | | | | | |
| Sample B (~1%)[7] | | | 5 | | | 2.5 | | 5 | | | | |
| C980[5] | | | | 0.6 | 0.3 | 0.3 | | | | | | |
| SF-1 (28.5%)[6] | | | | | | | | | | | | 5 |
| MCF (Cosun) (~1%)[8] | | | | | | | | | 5 | | 5 | |
| CMC | | | | | | | | | | 0.005 | 0.005 | |
| Citric Acid (50%) | 0.58 | 0.65 | 0.63 | 0.62 | 0.6 | 0.66 | 0.55 | 0.57 | 0.6 | 0.63 | 0.67 | 0.8 |
| pH | 4.55 | 4.47 | 4.49 | 4.44 | 4.56 | 4.5 | 4.6 | 4.6 | 4.51 | 4.53 | 4.6 | 4.44 |
| DI water (QS to) | 100 | 100 | 100 | 100 | 100 | 100 | 100 | 100 | 100 | 100 | 100 | 100 |

(continued)

| Sample Label | Control | Sample A | Sample B | C980 | Sample A/ C980 | Sample B/ C980 | Sample A (w/o C13s) | Sample B (w/o C13s) | Cosun MCF | CMC | Cosun MCF + CMC | SF-1 |
|---|---|---|---|---|---|---|---|---|---|---|---|---|
| BV Viscosity (22.5°C, RV5 @20rpm) | 13040 | 15700 | 14250 | 16550 | 12450 | 15550 | 10480 | 10860 | 13220 | 13060 | 13440 | 33700 |

[1]Guar Hydroxypropyltrimonium Chloride, Solvay
[2]Sodium Laureth Sulfate (1 mole of ethoxylation), Lubrizol Advanced Materials, Inc.
[3]Cocamidopropyl Betaine, Lubrizol Advanced Materials, Inc.
[4]Silicone Emulsion, Dow Corning
[5]Carbopol™ 980 Carbomer, Lubrizol Advanced Materials, Inc.
[6]Carbopol™ Aqua SF-1, Crosslinked Emulsion Polymer of (meth)acrylic acid and at least one $C_1$-$C_5$ alkyl (meth)acrylate, Lubrizol Advanced Materials, Inc.
[7]INCI: Cellulose (and) Cellulose Gum (and) Glycerin, C.P. Kelco
[8]Microfibrous Cellulose, Betafib™ MCF supplied by Royal Cosun - examples with MFC alone are not according to the invention, only examples with FDC are according to the invention.

## Claims

1. A cleansing composition comprising:

    a) detersive surfactant;
    b) a cationic polymer;
    c) a silicone conditioning agent; and
    d) a cellulosic material selected from fermentation derived cellulose (FDC).

2. A cleansing composition of claim 1 further comprising xanthan gum, guar gum, carboxymethylcellulose, and mixtures thereof.

3. A cleansing composition of claim 2, wherein the weight ratio of FDC to xanthan gum and carboxymethylcellulose is 6:3:1, respectively.

4. A cleansing composition of claim 2, wherein the weight ratio of FDC to guar gum and carboxymethylcellulose is 3:1:1, respectively.

5. A method for enhancing silicone deposition on keratinous substrates from a cleansing composition comprising at least one detersive surfactant, at least one cationic polymer, at least one silicone conditioning agent, said method including a step of incorporating a cellulosic material selected from fermentation derived cellulose.

## Patentansprüche

1. Reinigungszusammensetzung, umfassend:

    a) detersives Tensid;
    b) ein kationisches Polymer;
    c) ein Silikon-Konditioniermittel; und
    d) ein Cellulosematerial, das aus durch Fermentation gewonnener Cellulose (FDC) ausgewählt ist.

2. Reinigungszusammensetzung nach Anspruch 1, ferner umfassend Xanthangummi, Guargummi, Carboxymethylcellulose und Mischungen davon.

3. Reinigungszusammensetzung nach Anspruch 2, wobei das Gewichtsverhältnis von FDC zu Xanthangummi und Carboxymethylcellulose jeweils 6 : 3 : 1 ist.

4. Reinigungszusammensetzung nach Anspruch 2, wobei das Gewichtsverhältnis von FDC zu Guargummi beziehungsweise Carboxymethylcellulose 3 : 1 : 1 ist.

5. Verfahren zum Verbessern einer Silikonabscheidung auf keratinösen Substraten aus einer Reinigungszusammensetzung, umfassend mindestens ein detersives Tensid, mindestens ein kationisches Polymer, mindestens ein Silikon-Konditioniermittel, wobei das Verfahren einen Schritt eines Einarbeitens eines Cellulosematerials, das aus durch Fermentation gewonnener Cellulose ausgewählt ist, einschließt.

## Revendications

1. Composition nettoyante comprenant :

    a) un agent tensioactif détersif ;
    b) un polymère cationique ;
    c) un agent de conditionnement siliconé ; et
    d) un matériau cellulosique choisi parmi une cellulose dérivée de fermentation (FDC).

2. Composition nettoyante selon la revendication 1 comprenant en outre de la gomme de xanthane, de la gomme de guar, de la carboxyméthylcellulose, et des mélanges de celles-ci.

3. Composition nettoyante selon la revendication 2, dans laquelle le rapport pondéral de la FDC à la gomme de xanthane et à la carboxyméthylcellulose est de 6:3:1, respectivement.

4. Composition nettoyante selon la revendication 2, dans laquelle le rapport pondéral de la FDC à la gomme de guar et à la carboxyméthylcellulose est de 3:1:1, respectivement.

5. Procédé permettant d'améliorer un dépôt de silicone sur des substrats kératiniques à partir d'une composition nettoyante comprenant au moins un agent tensioactif détersif, au moins un polymère cationique, au moins un agent de conditionnement siliconé, ledit procédé comportant une étape d'incorporation d'un matériau cellulosique choisi parmi de la cellulose dérivée de fermentation.

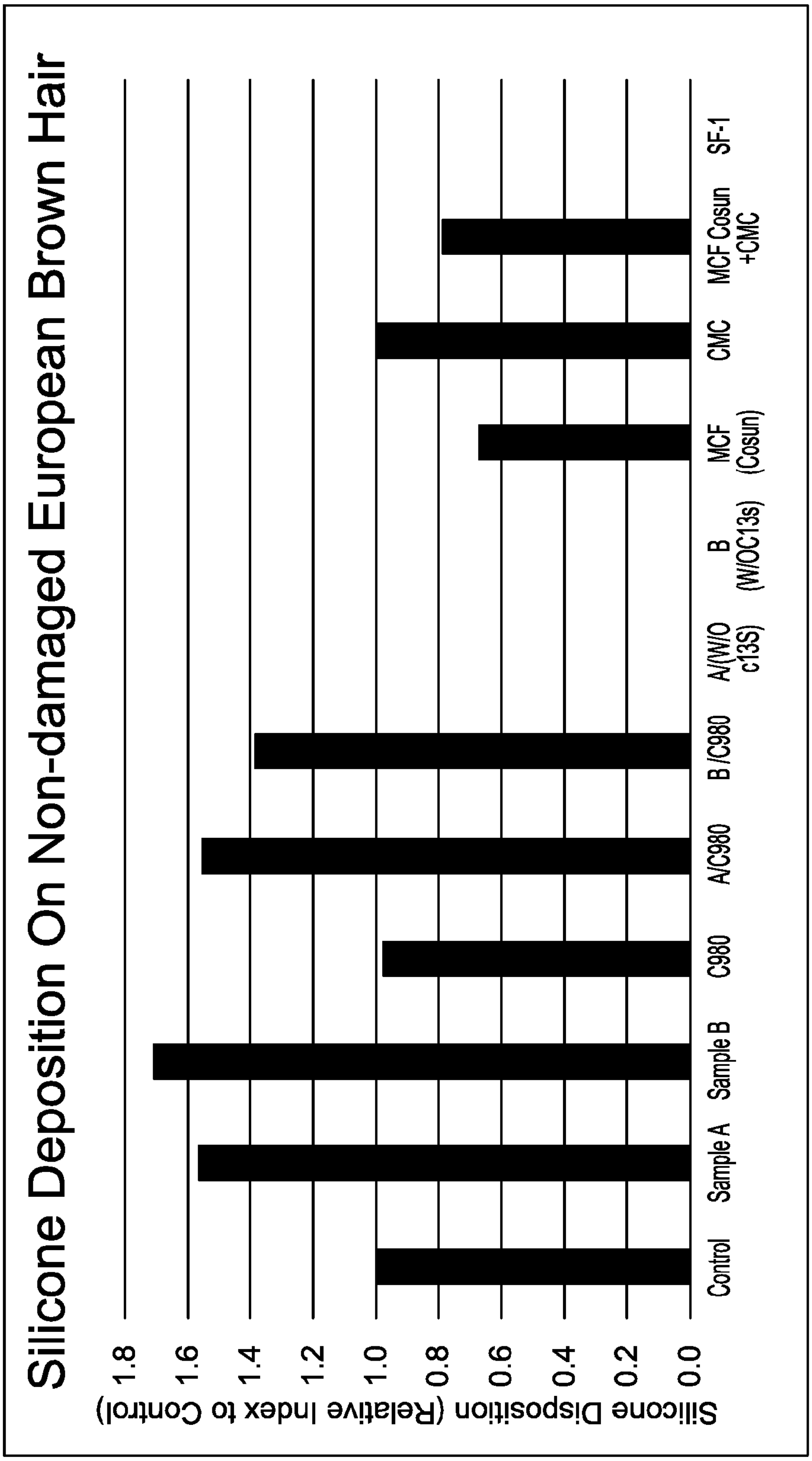
Silicone Deposition On Non-damaged European Brown Hair
Silicone Disposition (Relative Index to Control)
1.8
1.6
1.4
1.2
1.0
0.8
0.6
0.4
0.2
0.0
Control
Sample A
Sample B
C980
A/C980
B /C980
A/(W/O c13S)
B (W/OC13s)
MCF (Cosun)
CMC
MCF Cosun +CMC
SF-1

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10214708 B2
- US 20160317424 A
- US 6200554 B
- US 5136063 A
- US 5180843 A
- US 5098979 A
- US 5166297 A
- US 4285841 A, Barrat
- US 4284532 A, Leikhim
- US 5527892 A
- US 5770543 A
- US 6573375 B
- US 6727357 B
- US 3657175 A
- US 4384096 A
- US 4464524 A
- US 4801671 A
- US 5292843 A
- US 3835057 A
- US 4714653 A
- US 6488943 B
- US 4654207 A
- US 5019376 A


### Non-patent literature cited in the description


- Encyclopedia of Polymer Science and Engineering. John Wiley & Sons, Inc, 1989, vol. 15, 204-308
- Silicones: Preparation, Properties and Performance. Dow Corning Corporation, 2005
- **TODD ; BYERS.** Volatile Silicone Fluids for Cosmetics. *Cosmetics and Toiletries,* 1976, vol. 91 (1), 27-32
- **KASPRZAK.** Volatile Silicones. *Soap/Cosmetics/Chemical Specialties,* December 1986, 40-43
- McCutcheon's Detergents and Emulsifiers. Allured Publishing Corporation, 1998
- **MCCUTCHEON.** Functional Materials. 1992
- McCutcheon's Detergents and Emulsifiers. 1998
- Kirk-Othmer, Encyclopedia of Chemical Technology. vol. 23, 478-541
- Alkali-Swellable and Alkali-Soluble Thickener Technology A Review. **GREGORY D. SHAY.** Polymers in Aqueous Media - Performance Through Association, Advances in Chemistry Series. Division Polymeric Materials, 1989, 457-494
- **HUNTING.** Opacifiers and pearling agents in shampoos. *Cosmetic and Toiletries,* July 1981, vol. 96, 65-78